**Europäisches Patentamt**

**European Patent Office**

**Office européen des brevets**

(19)

(11) Veröffentlichungsnummer: **0 173 876**
**B1**

# EUROPÄISCHE PATENTSCHRIFT

(12)

(45) Veröffentlichungstag der Patentschrift:
**21.06.89**

(21) Anmeldenummer: **85109941.6**

(22) Anmeldetag: **07.08.85**

(51) Int. Cl.⁴: **A 61 L 2/02**, A 61 L 2/24

(54) **Verfahren und Vorrichtung zur Reinigung, Desinfektion und Sterilisation von ärztlichen, insbesondere zahnärztlichen Instrumenten.**

(30) Priorität: **20.08.84 DE 3430605**

(43) Veröffentlichungstag der Anmeldung:
**12.03.86 Patentblatt 86/11**

(45) Bekanntmachung des Hinweises auf die Patenterteilung:
**21.06.89 Patentblatt 89/25**

(84) Benannte Vertragsstaaten:
**AT CH DE FR IT LI**

(56) Entgegenhaltungen:
**FR-A-2 332 074**
**GB-A-2 094 992**
**US-A-3 007 478**
**US-A-3 334 035**
**US-A-3 640 295**
**US-A-3 975 246**
**US-A-4 202 740**

(73) Patentinhaber: **Siemens Aktiengesellschaft Berlin und München, Wittelsbacherplatz 2, D-8000 München 2 (DE)**

(72) Erfinder: **Hohmann, Eugen, Leimenberg 32, D-6140 Bensheim (DE)**
Erfinder: **Mund, Konrad, Dr., Langenbrucker Weg 10, D-8525 Uttenreuth (DE)**
Erfinder: **Weidlich, Erhard, Dr., Am Tennenbach 41, D-8521 Spardorf (DE)**

## Beschreibung

Aus hygienischer Sicht sollen die bei ärztlicher bzw. zahnärztlicher Behandlung benutzten Instrumente, wie Zangen, Pinzetten, Bohrwerkzeuge etc., keimfrei sein. Um diese Forderung zu erfüllen, ist es in der Praxis üblich, die Instrumente nach Gebrauch mittels Ultraschallreinigungsanlagen (US-3 007 478 und 3 640 295) zu reinigen und in weiteren Arbeitsgängen zu desinfizieren bzw. zu sterilisieren. Die Desinfektion wird in der Regel mit chemischen Mitteln (Alkoholen, Phenolen) durchgeführt, während zur Sterilisation Heißluft von etwa 180 bis 200° C oder gespannter Heißdampf Verwendung finden.

Insbesondere Instrumente (oder auch Teile davon) aus nichtmetallischen Werkstoffen halten diesen relativ hohen thermischen Beanspruchungen beim Sterilisieren nicht stand. Um auch derartig empfindliche Instrumente schonend zu sterilisieren, sind sogenannte Gassterilisatoren auf der Basis von Äthylenoxid im Einsatz. Nachteilig bei dieser Methode sind die relativ lange Sterilisationszeit von einigen Stunden und, weil Äthylenoxid stark toxisch und feuergefährlich ist, der damit verbundene, relativ hohe sicherheitstechnische Aufwand.

Der in den Ansprüchen 1 und 7 angegebenen Erfindung liegt die Aufgabe zugrunde, die Nachteile der bekannten Methoden zu vermeiden und ein Verfahren und eine Vorrichtung anzugeben, mit der die Instrumente in relativ kurzer Zeit aus hygienischer Sicht einwandfrei, dennoch aber instrumenten- und umweltschonend gereinigt und gleichsam sterilisiert werden können.

Ein wesentlicher Vorzug der vorgeschlagenen Maßnahmen ist, daß die mit Keimen kontaminierten Instrumente nun nicht mehr in mehreren Arbeitsgängen zunächst desinfiziert (um eine Infektion des Bedienpersonals zu verhindern), anschließend manuell oder in einer Waschmaschine gereinigt und schließlich in einem weiteren Arbeitegang sterilisiert werden müssen. Gemäß der Erfindung können diese Schritte nunmehr selbsttätig in einer einzigen Apparatur in Folgender Reihenfolge durchgeführt werden:

1. Grobreinigung in einem Ultraschallbad.
2. Feinreinigung und Sterilisation im Ultraschallbad unter Umwälzung der Flüssigkeit über eine elektrolytische Zelle.
3. Spülen mit Sterilflüssigkeit im Ultraschallbad.
4. Trocknen mittels steriler Warmluft.

Nachfolgend wird das Verfahren sowie eine Vorrichtung nach diesem Verfahren näher beschrieben. Es zeigen:

Figur 1    die erfindungsgemäße Vorrichtung in einer schaubildlichen Darstellung,

Figur 2    eine Prinzipskizze der Vorrichtung nach Figur 1 mit der zugehörigen hydraulischen Schaltung,

Figur 3    einen Längsschnitt durch die in Figur 2 schematisch dargestellte elektrolytische Zelle,

Figur 4    eine Prinzipschaltung der gesamten Anlage,

Figuren 5    eine weitere Ausführungsform einer
und 6    elektrolytischen Zelle.

### 1. Beschreibung der Vorrichtung:

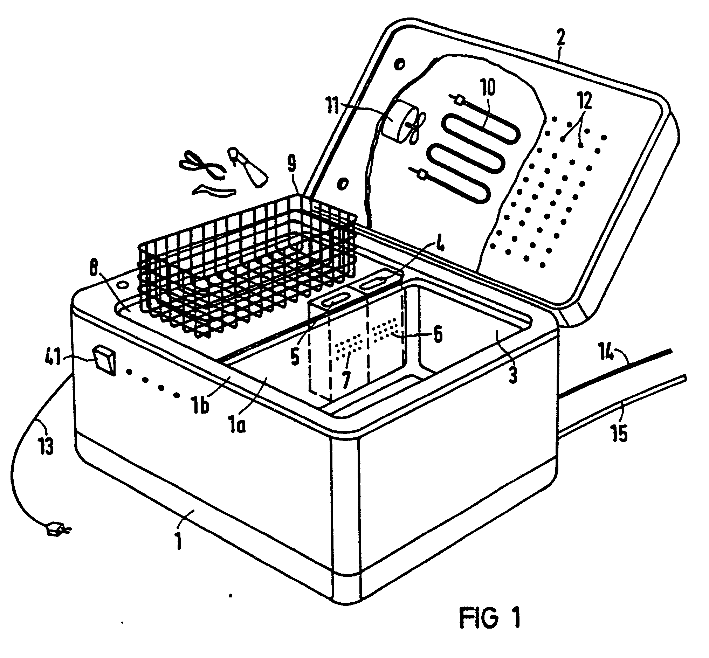

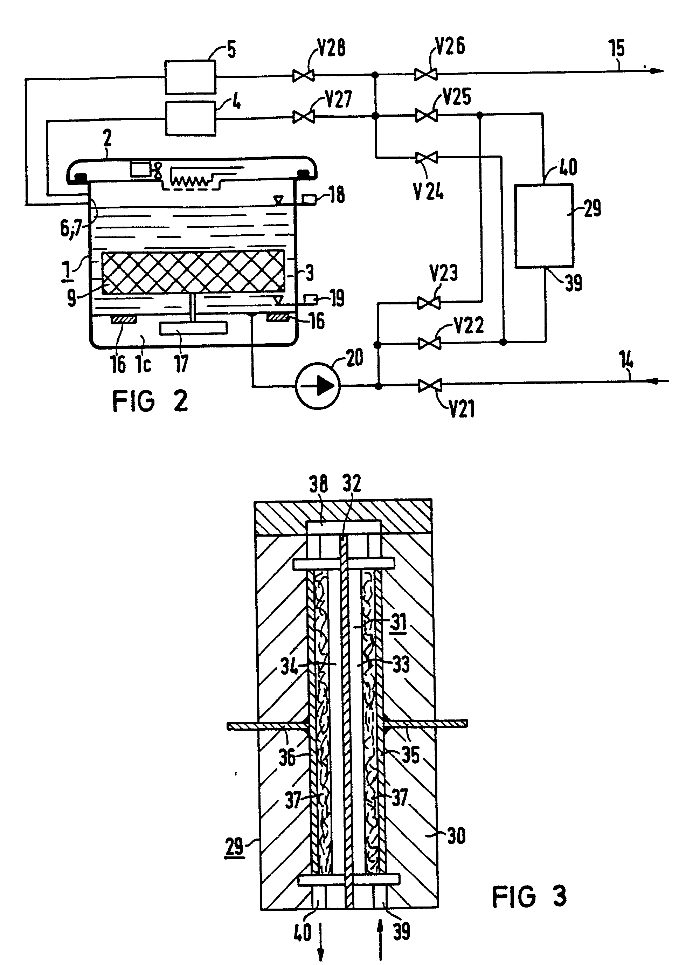

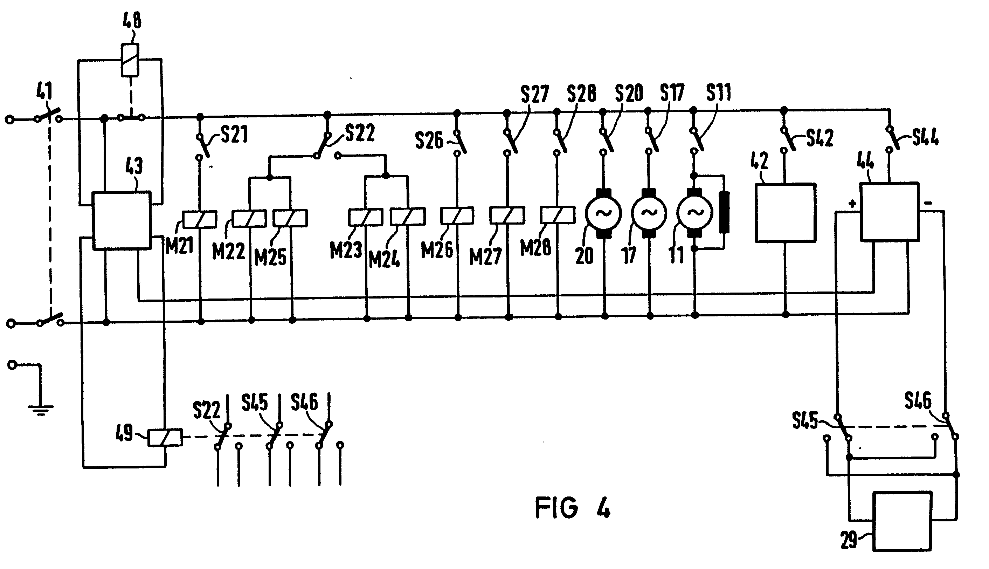

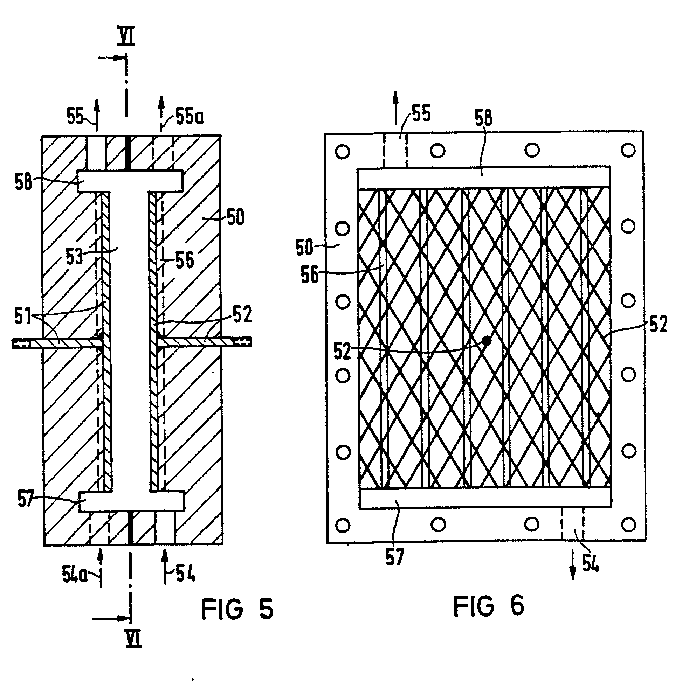

Die Figur 1 zeigt in einer schaubildlichen Darstellung eine nach dem nachfolgend noch näher beschriebenen Verfahren arbeitende Vorrichtung. Ein kastenförmig ausgebildetes, in zwei nebeneinanderliegende Gehäuseabschnitte 1a, 1b unterteiltes Gehäuse 1, welches mittels eines scharnierartig befestigten Deckels 2 dicht abgeschlossen werden kann, enthält im Gehäuseabschnitt 1a einen wannenartigen Behälter 3. Im Gehäuseabschnitt 1b ist ein Behälter 4 zur Aufnahme eines Reinigungsmittels (Tensid) sowie ein weiterer Behälter 5 zur Aufnahme eines Gemisches aus Natriumchlorid (NaCl) und eines Tensides untergebracht. Die beiden Behälter 4, 5 sind durch Öffnungen 6, 7 mit der Wanne 3 verbunden. Im Gehäuseabschnitt 1b sind weiterhin noch diverse, in Figur 2 und 4 aufgezeigte elektrische und hydraulische Bauteile sowie eine in Figur 5 näher dargestellte elektrolytische Zelle untergebracht. Der Gehäuseabschnitt 1b bildet deckseitig eine Ablage 8, auf der ein Drahtkorb 9 abgestellt werden kann, in den die zu reinigenden und zu sterilisierenden Instrumente (Scheren, Pinzetten, Bohrwerkzeuge etc.) eingelegt werden. In den ebenfalls kastenförmig ausgebildeten Deckel 2 sind eine Heizspirale 10 (z. B. Infrarotstrahler) sowie ein Miniaturlüfter 11 untergebracht. Mittels des Lüfters 11 kann, wie später noch erläutert, von der Heizspirale 10 erwärmte Luft über im Deckel vorgesehene Öffnungen 12 in die Wanne 3 geleitet werden. Die elektrische Versorgung der elektrischen Bauteile erfolgt über einen Netzanschluß 13. Mittels der Leitung 14 wird Frischwasser zugeführt, mittels der Leitung 15 Abwasser abgeführt.

In Betrachtung von Figur 2 wird deutlich, daß in einem unteren Gehäuseabschnitt 1c unmittelbar unterhalb des Behälters 3 und mit dessen Boden mechanisch gekoppelt ein in bekannter Weise aufgebauter Ultraschallschwinger 16 befestigt ist, über den die im Behälter 3 eingefüllte Flüssigkeit mit Ultraschallenergie beaufschlagt werden kann. Im Gehäuseabschnitt 1c ist ferner noch eine mit 17 bezeichnete Rütteleinrichtung angeordnet, ein an sich ebenfalls bekanntes Bewegungsaggregat, welches dazu dient, den in den Behälter eingesetzten Korb 9 bei Bedarf in eine Rüttelbewegung zu versetzen. Im Behälter 3 sind ferner zwei Füllstandsmesser, z. B. in Form von elektronischen Leitwertsonden, vorgesehen, ein oberer Füllstandsmesser 18 und ein unterer Füllstandsmesser 19. Mit 20 ist eine Umwälzpumpe bezeichnet, die, ebenso wie Magnetventile V21 bis V28 und eine mit 29 bezeichnete elektrolytische Zelle, im Gehäuseabschnitt 1b untergebracht sind.

Der Aufbau der elektrolytischen Zelle 29 wird anhand der Figuren 3, 5 und 6 näher erläutert. Bei der Zelle gemäß Figur 3 bildet ein im Querschnitt rechteckiges Gehäuse 30 aus Isoliermaterial mit den Maßen von ca. 150 x 100 x 30 mm einen Hohlraum 31, welcher durch eine längs eingespannte, ionenleitende Membran 32 in einen Anolytraum 33 und einen Katholytraum 34 unterteilt ist. Die Membran besteht vorzugsweise aus sulfoniertem Polytetrafluoräthylen und kann als Kationenaustauschermembran bezeichnet werden. Im Anolytraum 33 ist eine Anode 35, im Katholytraum 34 eine Kathode 36 angeordnet. Anode und Kathode bestehen aus gleichem Material, vorzugsweise aus aktiviertem Titan. Sie können auch aus Platinblech mit einem oder mehreren aufgepunkteten Netzen aus Platin oder aus Platin und Iridium bestehen. Anstelle der Netze können, wie in der Ausführung gemäß Figuren 5 und 6 gezeigt, auch Streckmetalle aus Titan oder Tantal eingesetzt werden, die mit Platinmohr oder Platin- und Rutheniumoxid belegt sind. Bei Anwendung solcher Elektroden kann auf die Trennembran zwischen Anode und Kathode verzichtet werden.

Vorteilhaft ist es auch, gegen Chlor beständige poröse Metalle, wie Raney-Platin und porösen Titanschwamm, oder gesinterte Metalle, die gegebenenfalls mit Edelmetallkatlaysatoren belegt sind, als Elektrodenmaterial einzusetzen.

Mit 37 ist ein Turbulenzgitter bezeichnet, welches in den beiden Räumen 33, 34 eingebracht ist und dazu dienen soll, die Wasserströmung in einen Turbulenzzustand zu versetzen. Als Material ist zweckmäßigerweise ein chemikalienbeständiges Gewebe, z. B. aus Teflon, vorgesehen. Bei Verwendung von Streckmetall-Elektroden, wie in Figuren 5 und 6 gezeigt, kann das Turbulenzgitter entfallen. Die notwendige Verwirbelung des durchfließenden Wassers wird bereits durch den geometrischen Aufbau des Streckmetalles erreicht.

Der Anolytraum 33 ist über einen Kanal 38 mit dem Katholytraum 34 verbunden, wodurch die in Pfeilrichtung über den Kanal 39 einströmende Flüssigkeit nach Durchgang durch den Anolytraum in den Katholytraum übertreten und nach dessen Durchströmen am Auslaßkanal 40 wieder austreten kann.

Eine andere vorteilhafte Ausführungsform einer elektrolytischen Zelle ist in den Figuren 5 und 6 dargestellt. Die äußere Form der Zelle und der Elektrodenaufbau sind im wesentlichen wie in Figur 3, d. h. in einem Gehäuse 50 aus Isoliematerial sind in einem Abstand von 0,5 bis 5 mm zwei an Gleichspannung anlegbare Elektroden 51, 52 angeordnet. Im Gegensatz zu der zuvor beschriebenen Ausführung ist jedoch keine Membran vorhanden, die den Hohl- bzw. Durchflußraum 53 unterteilen würde. Demgemäß sind Zuflußkanal 54 und Abflußkanal 55 einander gegenüberstehend angeordnet. Um einen gleichmäßigeren Durchfluß der Flüssigkeit durch die Zelle zu bekommen, ist es zweckmäßig, wie gestrichelt eingezeichnet, mehrere Zu- und Abflußkanäle 54a, 55a vorzusehen. Zur Verbesserung des Wirkungsgrades ist es außerdem vorteilhaft, zwischen Elektroden und Gehäuse Durchflutungs- bzw. Entlüftungskanäle 56 vorzusehen, die, wie aus der Schnittdarstellung in Figur 6 ersichtlich, in Durchflußrichtung angeordnet sind. Die Kanäle können ebenso wabenförmig ausgebildet sein; denkbar, und im Rahmen der Erfindung liegt es, auch, die Elektroden gegenüber dem Gehäuse durch andere geeignete Maßnahmen auf Abstand zu setzen. Vorteilhaft für einem optimalen Durchfluß ist es, außerdem vor und nach den Elektroden einen über die Breite der Elektroden sich erstreckenden Verteilerraum 57, 58 vorzusehen.

Wenngleich zwar der Wirkungsgrad der zuletzt beschriebenen Einkammer-Zelle nicht so gut ist, wie der der zuerst beschriebenen Zweikammer-Zelle, so ist doch der Aufbau der Einkammer-Zelle und insbesondere auch der hydraulische Schaltungsaufwand für diesen Zellentyp - wie später noch ausgeführt - erheblich einfacher. Bei Verwendung der Zweikammer-Zelle bei stark kalkhaltigem Wasser (Härtegrade >10° dH) empfiehlt es sich, um kalkablagerungen an der Trennwand zu vermeiden, der Anlage einen Ionenaustauscher voranzuschalten.

## 2. Beschreibung des Verfahrens:

Die kontaminierten Instrumente werden in den Drahtkorb 9 eingelegt und dieser in den zunächst leeren Reinigungsbehälter 3 eingesetzt. In die Behälter 4 und 5 wird entweder von Hand oder über externe Dosierspender, die in Nähe der Apparatur angebracht sein können, eine vordosierte Menge eines pulvrigen oder flüssigen Tensides in den einen und ein Gemisch aus Tensid und Natriumchlorid in den anderen Behälter eingefüllt. Nach Schließen des Deckels 2 kann der Reinigungs-, Desinfektions- und Sterilisationsprozeß beginnen.

Nach Drücken einer Starttaste 41 (Figur 1) wird über die Zuleitung 14 und die geöffneten Ventile 21, 22, 25 und 27 Frischwasser durch die elektrolytische Zelle 29 und durch den Tensidbehälter 4 in den Behälter 3 geleitet. Der Wasserstrom spült hierbei das im Tensidbehälter befindliche Reinigungsmittel in den Behälter ein. Nach Erreichen der notwendigen Füllhöhe, die durch den Füllstandsmesser 18 registriert wird, wird die Wasserzufuhr durch Schließen der Ventile 21 und 27 unterbrochen. Mit dem Startbefehl wird gleichzeitig das Bewegungsaggregat 17 eingeschaltet. Das Bewegungsaggregat versetzt den Korb 9 und die darin enthaltenen Instrumente in eine Rüttelbewegung, die eine vertikal- bzw. horizontaloszilierende Bewegung sein kann.

Mit dem Einschalten der Ultraschallelektronik 42 mittels Schalter S42 (Figur 4) wird der Ultraschallschwinger 16 (Figur 2) in Schwingungen versetzt, wodurch die im Behälter 3 befindliche Flüssigkeit mit Ultraschallenergie beaufschlagt wird. Damit beginnt die Grobreinigung. Für die Reinigungswirkung hat sich als günstig erwiesen, wenn die Ultraschallfrequenz unterhalb 35 kHz (etwa bei 30 kHz) liegt und die HF-Leistung mindestens 80 W/l Badinhalt beträgt. Das verwendete Tensid sollte eine geringe Schaumwirkung aufweisen. Durch von Bewegungsaggregat 17 erzeugte Bewegung wird die Reinigungswirkung erheblich unterstützt. Die erforderliche Reinigungszeit für die Grobreinigung beträgt etwa 3 bis 5 Min. (T1). Nach Abschalten der Ultraschallelektronik 42 wird der Inhalt des Behälters 3 in den Abfluß gepumpt. Hierzu wird die Umwälzpumpe 20 und das Magnetventil 26 eingeschaltet. Damit die durch die Reinigung von den Instrumenten abgespülten Keime nicht direkt in den Abfluß eingeleitet werden, ist es vorteilhaft, die Badflüssigkeit durch die geöffneten Ventile 22 und 25 und die inzwischen an Spannung liegende elektrolytische Zelle 29 hindurchzupumpen. Bei einer Stromdichte, die im Bereich von 50 mA/cm$^2$ bis 100 mA/cm$^2$ Elektrodenfläche liegt, erfolgt so eine elektrolytische Dissoziation, die ein Großteil der in der Flüssigkeit enthaltenen Keime abtötet. Ist der untere Füllstand erreicht, was durch den Füllstandsmesser 19 angezeigt wird, werden die elektrolytische Zelle 29, die Umwälzpumpe 20 und das Magnetventil 26 abgeschaltet.

Nunmehr beginnt erneut ein Füllvorgang, wobei anstelle des Magnetventils 27 das Magnetventil 28 geöffnet wird und der Wasserstrom den ein Gemisch von Natriumchlorid und Tensid enthaltenden Behälter 5 durchfließt und dessen Inhalt in den Behälter 5 mitreißt. Ist der Behälter 3 gefüllt, wird das Magnetventil 21 geschlossen.

Nunmehr beginnt der Verfahrensschritt der Feinreinigung und Sterilisation. Hierbei wird bei erneut eingeschalteter Ultraschallelektronik 42, an Spannung liegender Zelle 29, geöffneten Magnetventilen 22, 25 und 28 sowie eingeschalteter Umwälzpumpe 20 der Badinhalt eine bestimmte Zeit T2, die ebenfalls nur einige Minuten beträgt, kontinuierlich durch die elektrolytische Zelle umgewälzt. In dieser Umwälzung liegt die Besonderheit des Verfahrens. Die in der durchlaufenden Flüssigkeit enthaltenen Keime werden nämlich im Anolytraum der Zelle abgetötet. Der Abtötungsvorgang beruht auf Effekten, welche durch die elektrolytische Dissoziation hervorgerufen werden. Im einzelnen sind das
- direkte Oxydation der Keime durch Elektronenentzug bei Kontakt mit der Anodenfläche der Elektrode,
- indirekte Oxydation der Keime durch naszierenden Sauerstoff,
- Oxydation der Keime durch freies Chlor,
- Denaturierung der Keime durch den Säuregehalt der Lösung bei pH-Werten im Bereich von pH ~ 2.

Wie Versuche gezeigt haben, wurde eine mit 10$^6$ Keime/ml der Spezies bac. subtilis in ausgesporter Form beladene physiologische Kochsalzlösung in weniger als 3 Min. keimfrei gebracht. Mit Sporenerde (10$^4$ Keime/ml) geimpfte physiologische Kochsalzlösung war nach 5 Min. steril. Dabei lagen folgende Betriebsbedingungen zugrunde:

Badinhalt 1,5 l
Umwälzung 609 ml/min
Elektrodenfläche 100 cm$^2$
Stromdichte 100 mA/cm$^2$

Nach Durchströmen des Anolytraumes gelangt die Flüssigkeit in den Katholytraum. Durch Alkalisierung in Verbindung mit Wasserstoffentwicklung wird bei hartem Wasser das Calziumkarbonat abgeschieden, ein Nebeneffekt der die Feinreinigung begünstigt.

Nach Ablauf der Feinreinigung und Sterilisation, d.h. nach Abschalten der Ultraschallelektronik 42 und des Magnetventils 28, erfolgt in der gleichen, zuvor beschriebenen Weise das Entleeren des Behälters in den Abfluß und das erneute Füllen aus dem Leitungsnetz.

In der nachfolgenden Spülphase läuft der Behälterinhalt bei eingeschalteter Ultraschallelektronik 42 gleichfalls kontinuierlich unter Umwälzung durch die eingeschaltete elektrolytische Zelle 29. Die Zelle wird nunmehr jedoch mit einer reduzierten Stromdichte von etwa 5 mA/cm$^2$ bis 20 mA/cm$^2$ betrieben. Diese Stromdichte ist ausreichend, um eventuell durch das Netzwasser eingeschleppte Keime abzutöten. Die Spülphase erfolgt somit mit praktisch steriler Flüssigkeit. Nach Beendigung der Spülphase, die etwa 2 bis 3 Min. (T3) beträgt, wird der Behälterinhalt erneut durch Abpumpen in den Abfluß geleert.

Nunmehr erfolgt der Trocknungsvorgang. Hier wird durch die Heizwicklung 10 erzeugte und durch den Miniaturlüfter 11 über die Öffnungen 12 in den Behälter 3 eingeblasene Warmluft an das Sterilisiergut gebracht, wobei zweckmäßigerweise während des Trocknungsvorgangs das Bewegungsaggregat 17 eingeschaltet bleibt. Auch dieser Trocknungsvorgang beschränkt sich auf nur wenige Minuten (T4).

Die gesamte Steuerung des Verfahrensablaufes kann gemäß der Prinzipschaltung nach Figur 4 erfolgen, wobei eine Kontrollelektronik 43, welche vorzugsweise durch einen Mikroprozessor gebildet sein kann, den Programmablauf steuert. Als Eingangsgrößen dienen die Signale, welche aus den Füllstandsmessern 18 und 19 gewonnen werden. Verwendet man als Füllstandsmesser elektronische Leitwertsonden, so kann man aus dem elektrischen Leitwert der Flüssigkeit nicht nur die Steuerung des Füllstandes ableiten, sondern auch eine Kontrolle, ob die für eine Reinigung notwendigen Mengen an Tensid- bzw. Natriumchlorid vorhanden sind. Die Tensid- und die Natriumchloriddosierung kann auch aus der Zellspannung der elektrolytischen Zelle 29 und dem Zellstrom abgeleitet werden.

Die elektrolytische Zelle 29 bekommt Ihre Spannung aus einer mit 44 bezeichneten und über Schalter S44 eingeschalteten Steuerelektronik.

Im Störungsfall, d. h. wenn die vorgegebenen Grenzwerte der Eingangsgrößen überschritten werden, unterbricht ein Relais 48 den ablaufenden Prozeß und macht z. B. durch ein akustisches Signal eine optische Störanzeige auf Funktions- und Bedienungsfehler aufmerksam.

Nach jedem Programmablauf erfolgt mittels eines Relais 49 ein Umschalten der Kontakte S22, S45 und S46. Bei z. B. ungeradzahligen Programmabläufen kann das Relais 49 angezogen, bei geradzahligen Programmabläufen in Ruhestellung sein. Durch diese Umschaltung wird einerseits eine Umpolung der Strömungsrichtung durch die Zelle 29 (mittels Ventile 23, 24) und andererseits ein Polaritätswechsel an den Elektroden 35, 36 (mittels Umschaltkontakte S45, S46) erreicht. Nachdem, wie im Prinzipaufbau der elektrolytischen Zelle gemäß Figur 3 erläutert, die einfließende Flüssigkeit zuerst Kontakt bekommt mit der Anode, wird auch bei Umpolung und Umkehr der Strömungsrichtung die einfließende Flüssigkeit zuerst mit der Anode in Kontakt stehen. Dieser Vorgang ist insbesondere bei Betrieb mit hartem Wasser vorteilhaft, da das Kalziumkarbonat nicht nur in die Flüssigkeit ausfällt, sondern sich auch als Schicht auf der Kathodenelektrode aufbaut. Durch die Umschaltung findet also eine Rückbildung statt, die auch als Regeneration der Zelle bezeichnet werden kann. Damit wird eine gleichbleibende Funktion der Zelle gewährleistet.

Die Zudosierung von Reinigungsmittel und Natriumchlorid kann über eine Dosiereinrichtung aus größeren Vorratsbehältnissen erfolgen. Vom elektrischen Aufbau lassen sich ebenfalls noch weitere Vereinfachungen treffen; so lassen sich die Magnetventile V22, V23, V24 und V25 zur Umschaltung der Flußrichtung der elektrolytischen Zelle durch Verwendung von vier Zwei-Wegeventilen in ihrer Anzahl reduzieren.

Wie bereits erwähnt, läßt sich bei Verwendung der Zelle gemäß Figuren 5 und 6 wegen des Wegfalls der Flußrichtungsumkehr der Aufwand für die Steuerung des Medienflusses erheblich vereinfachen. In Betrachtung des Prinzipschaltbildes nach Figur 2 ergibt sich, daß die Ventile V22 bis V25 sowie die Leitungsabschnitte zu und von den Ventilen V23 und V24 entfallen können. In Betrachtung des Prinzipschaltbildes nach Figur 4 ergibt sich, daß darüber hinaus noch der Schalter S22 entbehrlich ist. Die übrigen Schaltungsmaßnahmen, so auch die Polaritätsumkehr an den Elektroden, bleiben erhalten.

**Patentansprüche**

1. Verfahren zur Reinigung, Desinfektion und Sterilisation von ärztlichen, insbesondere zahnärztlichen, Instrumenten, gekennzeichnet durch folgende Verfahrensschritte:
   a) die Instrumente werden in einem mit Flüssigkeit gefüllten und mit Reinigungsmitteln versetzten Behälter (3) unter Beaufschlagung der Flüssigkeit mit Ultraschallenergie während einer Zeit T1 vorgereinigt,
   b) nach Entleeren der Flüssigkeit wird erneut Flüssigkeit unter Beigabe von Reinigungsmittel und Natriumchlorid (NaCl) in den Behälter gegeben und die Flüssigkeit während einer Zeit T2 unter erneuter Beaufschlagung mittels Ultraschall über eine an Spannung liegende, nach dem Prinzip der elektrolytischen Dissoziation arbeitenden elektrolytischen Zelle (29) umgewälzt,
   c) nach Entleeren der Flüssigkeit wird Spülflüssigkeit in den Behälter (3) gegeben und die Flüssigkeit während einer Zeit T3 erneut mittels Ultraschall beaufschlagt und über die elektrolytische Zelle (29) umgewälzt,
   d) nach Entleeren dieser Flüssigkeit werden die Instrumente während einer Zeit T4 im Behälter (3) mittels Warmluft getrocknet.

2. Verfahren nach Anspruch 1, dadurch gekennzeichnet, daß die Flüssigkeit bereits vor Einspeisung in den Behälter (3) durch die elektrolytische Zelle (29) geleitet wird.

3. Verfahren nach Anspruch 1 oder 2, dadurch gekennzeichnet, daß die nach Beendigung eines Reinigungs-/Spülvorganges aus dem Behälter (3) austretende Flüssigkeit ebenfalls über die elektrolytische Zelle (29) geleitet wird.

4. Verfahren nach einem der Ansprüche 1 bis 3, dadurch gekennzeichnet, daß die Flüssigkeit vor Eintritt in den Behälter (3) durch das Reinigungsmittel bzw. Reinigungsmittel und NaCl aufnehmende Behältnisse (4, 5) geleitet wird.

5. Verfahren nach einem der Ansprüche 1 bis 4, dadurch gekennzeichnet, daß während der Beaufschlagung der Behälterflüssigkeit mit Ultraschall der Behälter (3) oder ein im Behälter (3) angeordneter, die Instrumente aufnehmender weiterer Behälter (9) in eine Rüttelbewegung versetzt wird.

6. Verfahren nach einem der Ansprüche 1 bis 5, dadurch gekennzeichnet, daß der elektrische Leitwert der Flüssigkeit gemessen wird und daraus Steuersignale ermittelt werden, die an eine zentrale Steuereinheit, vorzugsweise an einen Mikroprozessor, gegeben werden, welcher die Verfahrensschritte selbsttätig ablaufen läßt.

7. Vorrichtung zur Durchführung des Verfahrens nach einem der Ansprüche 1 bis 6, dadurch gekennzeichnet, daß in einem gemeinsamen Gehäuse (1) ein Behälter (3) zur Aufnahme der Instrumente, eine elektrolytische Zelle (29), ein Ultraschallschwinger (16) eine Trockeneinrichtung (10) sowie elektrische und hydraulische Steuerglieder (21 bis 28, 40 bis 47) angeordnet sind.

8. Vorrichtung nach Anspruch 7, <u>dadurch gekennzeichnet</u>, daß das Gehäuse (1) unterteilt ist in einen ersten, den Behälter (3) beinhaltenden Gehäuseabschnitt (1a) und einen sich daran anschließenden, in sich abgeschlossenen Gehäuseabschnitt (1b), in dem die elektrischen und hydraulischen Steuerglieder (21 bis 28, 40 bis 47) angeordnet sind und daß in dem zweiten Gehäuseabschnitt (1b) Behältnisse (4, 5) zur Aufnahme von einerseits Reinigungsmittel und andererseits Reinigungsmittel und Natriumchlorid vorgesehen sind und die Behältnisse über Öffnungen (6, 7) mit dem Behälter (3) verbunden sind.

9. Vorrichtung nach einem der Ansprüche 7 oder 8, <u>gekennzeichnet durch</u> einen das Gehäuse (1) nach oben abschließenden Deckel (2), in dem eine Heizvorrichtung (10), vorzugsweise ein Infrarotstrahler, untergebracht ist.

10. Vorrichtung nach Anspruch 9, <u>dadurch gekennzeichnet</u>, daß im Deckel zusätzlich noch ein Lüfter (11) zur Umwälzung der mittels der Heizvorrichtung (10) erwärmten Luft vorgesehen ist.

11. Vorrichtung nach einem der Ansprüche 7 bis 10, <u>dadurch gekennzeichnet</u>, der Behälter (3) Füllstandsmesser (18, 19), vorzugsweise elektrische Leitwertsonden, zur Bestimmung eines oberen und eines unteren Füllstandpegels enthält.

12. Vorrichtung nach einem der Ansprüche 7 bis 11, <u>dadurch gekennzeichnet</u>, daß der Ultraschallschwinger (16) mit einer Frequenz von unterhalb 35 kHz und mit einer Leistung von wenigstens 80 W/l Behälterinhalt betrieben wird.

13. Vorrichtung nach einem der Ansprüche 7 bis 12, <u>dadurch gekennzeichnet</u>, daß ein Umschaltwerk (S22, S46, S45) vorhanden ist, mit dem nach Beendigung des Programmablaufs die Spannungspolarität an der elektrolytischen Zelle (29) und gegebenenfalls auch die Strömungsrichtung der Flüssigkeit durch die Zelle bis zum Beginn des nächsten Programms umgepolt werden.

14. Vorrichtung nach einem der Ansprüche 7 bis 13, <u>dadurch gekennzeichnet</u>, daß die elektrolytische Zelle (29) aus einem länglichen Körper (30) besteht, der eine Durchflußkammer (31) bildet, in der zwei eine Anode und eine Kathode bildende Elektroden (35, 36) in engem Abstand einander gegenüberstehen, daß eine ionendurchlässige Trennwand (32) zwischen den beiden Elektroden vorhanden ist, die den Hohlraum (31) in einen die Anode aufnehmenden Anolytraum (33) und in einen die Kathode aufnehmenden Katholytraum (34) unterteilt, daß Anoden- und Kathodenraum an der einen Stirnseite durch einen Strömungskanal (38) miteinander verbunden sind und an der anderen stirnseite je einen Anschluß (39, 40) aufweisen, wobei der der Anode benachbarte Anschluß den Eingang und der andere Anschluß (40) den Ausgang der Zelle bilden.

15. Vorrichtung nach Anspruch 14, <u>dadurch gekennzeichnet</u>, daß Anoden- und Kathodenraum (33, 34) der elektrolytischen Zelle (29) durch eine Kationenaustauschermembran (32) voneinander getrennt sind, deren Strömungswiderstand groß ist gegenüber demjenigen von Anoden- und Kathodenraum (33, 34).

16. Vorrichtung nach Anspruch 7, <u>dadurch gekennzeichnet</u>, daß die elektrolytische Zelle (29) aus einem länglichen Körper (50) besteht, der eine Durchflußkammer (53) bildet, in der zwei eine Anode und Kathode bildende Elektroden (51, 52) in engem Abstand einander gegenüberstehen, die an ihren der Durchflußkammer (53) abgewandten Seiten mit dem Körper (50) Durchflutungs- bzw. Lüftungskanäle (56) bilden.

17. Vorrichtung nach Anspruch 16, <u>dadurch gekennzeichnet</u>, daß die Elektroden (35, 36, 51, 52) aus aktiviertem Titan bestehen, vorzugsweise in Form eines Netzes oder Streckmetalles.

18. Vorrichtung nach Anspruch 17, <u>dadurch gekennzeichnet</u>, daß die Elektroden (35, 36) mit Edelmetallen aktiviert sind.

19. Vorrichtung nach Anspruch 16, <u>dadurch gekennzeichnet</u>, daß die Elektroden (35, 36) Elektrokatalysatoren enthalten.

20. Vorrichtung nach einem der Ansprüche 7 bis 19, <u>dadurch gekennzeichnet</u>, daß die Zelle (29) während der Reinigungsphase mit einer Stromdichte von 50 mA bis 100 ma/$cm^2$ Elektrodenfläche und während der Spülphase mit reduzierter, vorzugsweise zwischen 5 mA und 20 mA/$cm^2$ Elektrodenfläche betrieben wird.

21. Vorrichtung nach einem der Ansprüche 7 bis 20, <u>dadurch gekennzeichnet</u>, daß die wirksame Elektrodenfläche zwischen 50 und 250 $cm^2$ liegt, vorzugsweise 100 cm beträgt.

## Claims

1. Process for cleaning, disinfecting and sterilizing medical, more particularly dental instruments, characterized by the following procedural steps:

a) the instruments are pre-cleaned for a time T1 in a receptacle (3) filled with liquid and having the addition of cleaning means whilst the liquid is acted upon by ultrasound energy,

b) after the liquid is emptied, liquid is put anew into the receptacle with the addition of cleaning means and sodium chloride (NaCl) and the liquid is circulated for a time T2, with renewed action thereupon by means of ultrasound, over a cubicle (29), connected to the supply and operating according to the principle of the electrolytic dissociation,

c) after the liquid is emptied, rinsing liquid is put into the receptacle (3) and the liquid is acted upon anew for a time T3 by means of ultrasound and is circulated over the electrolytic cubicle (29),

d) after this liquid is emptied, the instruments are dried for a time T4 in the receptacle (3) by means of warm air.

2. Process according to claim 1, characterized in that the liquid is led through the electrolytic cubicle (29) just prior to being supplied into the receptacle (3).

3. Process according to claim 1 or 2, characterized in that the liquid issuing from the receptacle (3) after a cleaning/rinsing process has finished, is likewise led over the electrolytic cubicle (29).

4. Process according to one of claims 1 to 3, characterized in that prior to entering the receptacle (3), the liquid is led through the containers (4, 5) which hold the cleaning means or the cleaning means and NaCl.

5. Process according to one of claims 1 to 4, characterized in that while the liquid in the receptacle is being acted upon by ultrasound, the receptacle (3) or an additional receptacle (9), arranged in the receptacle (3) and holding the instruments, is set in a shaking motion.

6. Process according to one of claims 1 to 5, characterized in that the electric conductivity of the liquid is measured and control signals are determined therefrom which are given to a central control unit, preferably to a microprocessor which allows the procedural steps to occur automatically.

7. Device for carrying out the process according to one of claims 1 to 6, characterized in that a receptacle (3) for holding the instruments, an electrolytic cubicle (29), an ultrasound vibrator (16), a drying apparatus (10) as well as electric and hydraulic control elements (21 to 28, 40 to 47) are arranged in a common housing (1).

8. Device according to claim 7, characterized in that the housing (1) is subdivided into a first housing part (1a) containing the receptacle (3) and a housing part (1b) which is attached thereto and which is selfcontained, into which the electric and hydraulic control elements (21 to 28, 40 to 47) are arranged and in that in the second housing part (lb) containers (4, 5) are provided for holding cleaning means on the one hand and cleaning means and sodium chloride on the other hand and the containers are connected with the receptacle (3) by means of openings (6, 7).

9. Device according to one of claims 7 or 8, characterized by a cover (2) which shuts off the housing (1) at the top, in which cover there is installed a heating device (10), preferably an infrared radiator.

10. Device according to claim 9, characterized in that in addition a fan (11) is also provided in the cover in order to circulate the air warmed by means of the heating device (10).

11. Device according to one of claims 7 to 10, characterized in that the receptacle (3) contains liquid level measures (18, 19), preferably electric conductivity probes in order to determine an upper and a lower liquid level gauge.

12. Device according to one of claims 7 to 11, characterized in that the ultrasound oscillator (16) is operated with a frequency of under 35 kaz and with a power of at least 80 W/l receptacle content.

13. Device according to one of claims 7 to 12, characterized in that a change-over mechanism (S22, S46, S45) is present with which, after the course of the programme has finished, the voltage polarity at the electrolytic cubicle (29) and likewise also the flow direction of the liquid through the cubicle are reversed until the beginning of the next programme.

14. Device according to one of claims 7 to 13, characterized in that the electrolytic cubicle (29) consists of an oblong body (30) which forms a flow compartment (31) in which two electrodes (35, 36), forming an anode and a cathode, face each other in close proximity, in that, present between the two electrodes, there is a dividing wall (32), which is permeable with regard to ions, and which subdivides the hollow cavity (31) into an anolyte space (33) which holds the anode and into a catholyte space (34) which holds the cathode (36), in that the anode space and cathode space are connected with each other at the one front side by means of a flow channel (38) and at the other front side they each have a connection (39, 40) whereby the connection adjacent to the anode forms the entrance and the other connection (40) forms the exit of the cubicle.

15. Device according to claim 14, characterized in that the anode space and cathode space (33, 34) of the electrolytic cubicle (29) are separated from one another by a cation exchanger diaphragm (32), the flow resistance of which is great compared with that of the anode space and cathode space (33, 34).

16. Device according to claim 7, characterized in that the electrolytic cubicle (29) consists of an oblong body (50) which forms a flow compartment (53) in which two electrodes (51, 52), forming an anode and a cathode, face each other in close proximity, which form with the body (50) flow channels or ventilating channels (56) at their sides which are directed away from the flow compartment (53).

17. Device according to claim 16, characterized in that the electrodes (35, 36, 51, 52) consist of activated titanium, preferably in the form of a network or expanded metal.

18. Device according to claim 17, characterized in that the electrodes (35, 36) are activated with noble metals.

19. Device according to claim 16, characterized in that the electrodes (35, 36) contain electric catalysts.

20. Device according to one of claims 7 to 19, characterized in that the cubicle (29) is operated with a current density of 50 mA to 100 mA/cm$^2$ electrode surface during the cleaning phase and with a reduced current density, preferably between 5 mA and 20 mA/cm$^2$ electrode surface during the rinsing phase.

21. Device according to one of claims 7 to 20, characterized in that the effective electrode surface lies between 50 and 250 cm$^2$, preferably amounting to 100 cm$^2$.

## Revendications

1. Procédé pour nettoyer, désinfecter et stériliser des instruments médicaux, notamment des instruments de dentisterie, caractérisé par les étapes opératoires suivantes:

a) on réalise un nettoyage préalable des instruments pendant un intervalle de temps T1 dans un récipient (3), qui est rempli par un liquide et dans lequel sont ajoutés des agents de nettoyage, moyennant l'application d'une énergie ultrasonore au liquide,

b) après vidage du liquide, on introduit à nouveau un liquide dans le récipient en ajoutant un agent de nettoyage et du chlorure de sodium (NaCl) et on fait circuler le liquide pendant un intervalle de temps T2 en lui appliquant à nouveau une énergie ultrasonore, dans une cellule électrolytique (29) placée sous tension et fonctionnant selon le principe de la dissociation électrolytique,

c) après vidage du liquide, on introduit un liquide de lavage dans le récipient (3) et on fait circuler le liquide pendant un intervalle de temps T3 à nouveau en lui appliquant une énergie ultrasonore et en lui faisant traverser la cellule électrolytique (29),

d) après vidage de ce liquide, on fait sécher les instruments pendant un intervalle de temps T4 dans le récipient (3) en utilisant de l'air chaud.

2. Procédé suivant la revendication 1, caractérisé par le fait que dès avant l'introduction du liquide dans le récipient (3), on lui fait traverser la cellule électrolytique (29).

3. Procédé suivant la revendication 1 ou 2, caractérisé par le fait qu'on fait également traverser la cellule électrolytique (29) par le liquide sortant du récipient (3) à la fin de l'opération de nettoyage/lavage.

4. Procédé suivant l'une des revendications 1 à 3, caractérisé par le fait qu'on fait traverser les récipients 4,5 recevant les agents de nettoyage ou l'agent de nettoyage et le NaCl, par le liquide avant son introduction dans le récipient (3).

5. Procédé suivant l'une des revendications 1 à 4, caractérisé par le fait que pendant l'application des ultrasons au liquide contenu dans le récipient (3), on soumet ce récipient ou un autre récipient (9) disposé dans le récipient (3) et contenant les instruments à un mouvement de secouage.

6. Procédé suivant l'une des revendications 1 à 5, caractérisé par le fait qu'on mesure la conductivité électrique du liquide et qu'on détermine, à partir de cette valeur, des signaux de commande qui sont envoyés à une unité centrale de commande, de préférence à un microprocesseur, qui commande l'exécution automatique des étapes opératoires.

7. Dispositif pour la mise en oeuvre du procédé suivant l'une des revendications 1 à 6, caractérisé par le fait qu'un récipient (3) destiné à recevoir les instruments, une cellule électrolytique (29), un oscillateur à ultrasons (16), un séchoir (10) ainsi que des éléments de commande électriques et hydrauliques (21 à 28, 40 à 47) sont disposés dans une enceinte commune (1).

8. Dispositif suivant la revendication 7, caractérisé par le fait que l'enceinte (1) est subdivisée en une première section (1a) contenant le récipient (3), et en une section (1b), raccordée à la précédente et fermée sur elle-même, et dans laquelle les éléments de commande électriques et hydrauliques (21 à 28, 40 à 47) sont disposés, que des logements (4, 5) destinés à recevoir d'une part un agent de nettoyage et d'autre part un agent de nettoyage et du chlorure de sodium sont prévus dans la seconde section (1b) de l'enceinte et que les logements sont réunis au récipient (3) par l'intermédiaire d'ouvertures (6, 7).

9. Dispositif suivant l'une des revendications 7 ou 8, caractérisé par un couvercle (2) qui ferme l'enceinte (1) à sa partie supérieure et dans lequel est logé un dispositif de chauffage (10), de préférence un émétteur infrarouge.

10. Dispositif suivant la revendication 9, caractérisé par le fait qu'un ventilateur (11) servant à faire circuler l'air chauffé au moyen du dispositif de chauffage (10) est prévu en supplément dans le couvercle.

11. Dispositif suivant l'une des revendications 7 à 10, caractérisé par le fait que le récipient (3) contient des appareils (18,19) de mesure du niveau de remplissage, de préférence des sondes détectant la conductivité électrique, pour déterminer un niveau supérieur de remplissage et un niveau inférieur de remplissage.

12. Dispositif suivant l'une des revendications 7 à 11, caractérisé par le fait qu'on fait fonctionner l'oscillateur à ultrasons (16) avec une fréquence inférieure à 35 kHz et avec une puissance égale à au moins 80 W/l de contenance du récipient.

13. Dispositif suivant l'une des revendications 7 à 12, caractérisé par le fait qu'il est prévu un mécanisme de commutation (S22, S46, S45), à l'aide duquel, à la fin du déroulement du programme, la polarité de la tension appliquée à la cellule électrolytique (29) et éventuellement également la direction de l'écoulement du liquide à travers la cellule sont inversées jusqu'au début du programme immédiatement suivant.

14. Dispositif suivant l'une des revendications 7 à 13, caractérisé par le fait que la cellule électrolytique (29) est constituée par un corps allongé (30), qui forme une chambre à traversée (31), dans laquelle deux électrodes (35, 36) constituant une anode et une cathode sont disposées en vis-à-vis en étant séparées par une faible distance, qu'entre les deux électrodes il est prévu une paroi de séparation (32), qui est perméable aux ions et subdivise la cavité (31) en une chambre (33) pour l'anolyte, qui reçoit l'anode, et en une chambre (34) pour le catholyte, qui reçoit la cathode, que les chambres recevant l'anode et la cathode sont reliées entre elles, au niveau d'une de leurs faces frontales, par un canal d'écoulement (38) et comportent des raccords respectifs (39, 40) sur l'autre face frontale, le raccord voisin de l'anode formant l'entrée de la cellule et l'autre raccord (40) en constituant la sortie.

15. Dispositif suivant la revendication 14, caractérisé par le fait que les chambres (33, 34), qui logent l'anode et la cathode, de la cellule électrolytique (29) sont séparées par une membrane (32) échangeuse de cations, dont la résistance à l'écoulement est élevée par rapport à celle des chambres (33, 34) recevant l'anode et la cathode.

16. Dispositif suivant la revendication 7, caractérisé par le fait que la cellule électrolytique (29) est constituée par un corps allongé (50), qui forme une chambre de passage (53), dans laquelle deux électrodes (51, 52) formant une anode et une cathode sont disposées en vis-à-vis en étant séparées par une faible distance, les faces de ces électrodes, tournées à l'opposé de la chambre de passage (53) délimitant, avec le corps (50), des canaux d'écoulement ou d'aération (56).

17. Dispositif suivant la revendication 16, caractérisé par le fait que les électrodes (35, 36, 51, 52) sont constituées par du titane actif, de préférence sous la forme d'un treillis ou d'un métal déployé.

18. Dispositif suivant la revendication 17, caractérisé par le fait que les électrodes (35, 36) sont activées par des métaux précieux.

19. Dispositif selon la revendication 16, caractérisé par le fait que les électrodes (35, 36) contiennent des catalyseurs électriques.

20. Dispositif suivant l'une des revendications 7 à 19, caractérisé par le fait que, pendant la phase de nettoyage, on fait fonctionner la cellule (29) avec une densité de courant 50 mA à 100 mA/cm$^2$ de surface d'électrode et, pendant la phase de lavage, avec une densité de courant réduite, comprise de préférence entre 5 mA et 20 mA/cm$^2$.

21. Dispositif suivant les revendications 7 à 20, caractérisé par le fait que la surface active des électrodes est comprise entre 50 et 250 cm$^2$ et est égale de préférence à 100 cm$^2$.

FIG 1

FIG 2

FIG 3

FIG 4

FIG 5

FIG 6